(19) Europäisches Patentamt
European Patent Office
Office européen des brevets

(11) Publication number: 0 311 051
A2

(12) # EUROPEAN PATENT APPLICATION

(21) Application number: 88116494.1

(51) Int. Cl.4: C07D 213/57 , A01N 43/40

(22) Date of filing: 05.10.88

(30) Priority: 06.10.87 JP 250765/87

(43) Date of publication of application: 12.04.89 Bulletin 89/15

(84) Designated Contracting States: BE CH DE ES FR GB IT LI NL

(71) Applicant: SDS Biotech K.K.
12-7, Higashi Shinbashi 2-chome
Minato-ku Tokyo 105(JP)

(72) Inventor: Ishii, Teruhiko SDS Biotech K.K., Research Inst.
24-25, Tamagawa 2-chome
Ohta-ku Tokyo(JP)
Inventor: Motoyoshi, Masatoshi SDS Biotech K.K., Res. Inst.
24-25, Tamagawa 2-chome
Ohta-ku Tokyo(JP)
Inventor: Yamaguchi, Hiromi SDS Biotech K.K., Res. Inst.
24-25, Tamagawa 2-chome
Ohta-ku Tokyo(JP)

(74) Representative: Patentanwälte Grünecker, Kinkeldey, Stockmair & Partner
Maximilianstrasse 58
D-8000 München 22(DE)

(54) **Pyridylacetonitrile derivatives.**

(57) Pyridylacetonitrile derivatives, inclusive of acid salts and metal complexes thereof, of the general formula (I)

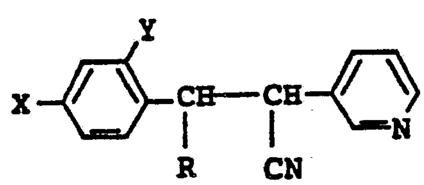

(I)

wherein X is a halogen atom or a phenylethynyl group, Y is a hydrogen atom, a halogen atom, a methyl group or a trifluoromethyl group, R is a lower alkyl group or a phenyl group which may be substituted or unsubstituted with a halogen atom. These novel pyridylacetonitrile compounds are suitable for use as fungicides or germicides.

EP 0 311 051 A2

# PYRIDYLACETONITRILE DERIVATIVES

## FIELD OF THE INVENTION

The present invention relates to novel pyridylacetonitrile derivatives, and nonmedical fungicides or germicides, especially agricultural fungicides, containing the pyridylacetonitrile derivatives as an effective ingredient.

## BACKGROUND OF THE INVENTION

It has been reported in U.S. Patent 3,196,158 that 3-(2-chlorophenyl)-2-(3-pyridyl)propionitrile inhibits a certain function of the adrenal cortex. This patent does not, however, disclose the use as an agricultural fungicide, and the compound is structurally different from the pyridylacetonitrile derivatives according to the present invention.

On the other hand, it has been reported in EP-A-104,619 that 3-(2,4-dichlorophenyl)-3-isobutylthio-2-(3-pyridyl)propionitrile can be used as a fungicide. The fungicidal activity thereof, however, is not satisfactory, especially against gray mold, and the type of chemical structure is different from the compounds of the present invention. It has been also reported in JP-A-61-167668 (The term "JP-A" as used herein means an "unexamined published Japanese patent application") that azolylacetonitrile derivatives can be used as an agricultural fungicide. However, the structure of the disclosed compounds is different from the compounds of the present invention, and the fungicidal activity thereof is not satisfactory for the practical use.

## SUMMARY OF INVENTION

An object of the present invention is to provide novel pyridylacetonitrile derivatives, and nonmedical fungicides or germicides, especially agricultural fungicides, containing the pyridylacetonitrile derivatives as an effective ingredient.

As a result of intensive investigation to achieve the above objects, it has now been found that the above and other objects can be accomplished by a pyridylacetonitrile derivative, inclusive of acid salts and metal complexes thereof, of the general formula (I)

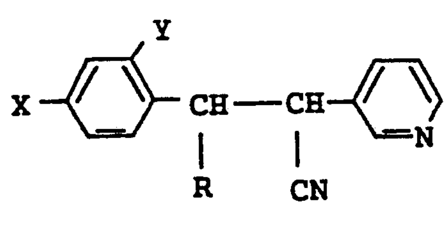

(I)

wherein X is a halogen atom or a phenylethynyl group, Y is a hydrogen atom, a halogen atom, a methyl group or a trifluoromethyl group, R is a lower alkyl group or a phenyl group which may be substituted or unsubstituted with a halogen atom.

In accordance with the present invention, there are also provided nonmedical fungicides or germicides comprising, as an effective ingredient, the pyridylacetonitrile derivatives represented by the general formula (I), or the physiologically acceptable salts or metal complexes thereof.

## DETAILED DESCRIPTION OF THE INVENTION

According to the present invention, there are provided novel pyridylacetonitrile derivatives represented by the above mentioned general formula (I). In the general formula (I), X represents a halogen atom or a phenylethynyl group, preferably chlorine, fluorine or phenylethynyl; Y represents a hydrogen atom, a halogen atom, a methyl group or a trifluoromethyl group, preferably a chlorine or fluorine atom; and R represents a lower alkyl group (preferably 1 to 6 carbon atoms) or a phenyl group which may be substituted or unsubstituted with a halogen atom (preferably chlorine or fluorine), more preferably alkyl group having 1 to 4 carbon atoms, most preferably a methyl group, an ethyl group or a n-propyl group.

The acid salts of the pyridylacetonitrile derivatives (I) are those which are formed from the compounds of the general formula (I) and inorganic or organic acids. The typical examples of the inorganic or organic acids are hydrochloric acid, hydrobromic acid, sulfuric acid, oxalic acid, sulfonic acid, and the like.

The metal complexes of the pyridylacetonitrile derivatives (I) are those which are composed of the compounds of the general formula (I) and metal salts. The preferred metal complexes can be represented by the following general formula (II)

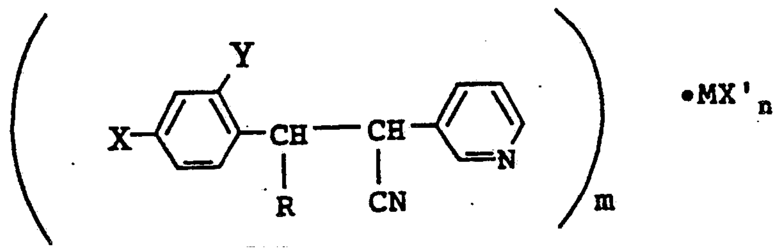

(II)

wherein X, Y and R are as defined above and M is a metal atom and $X'$ is a counter ion, and n and m each represents integers of 1 to 4. The preferred examples of M are aluminum, manganese, cobalt, iron, nickel, cupper or zinc, preferably cupper or zinc. The preferred examples of $X'$ are chloride, bromide, iodide, sulfate, phosphate, nitrate, carbonate or acetate ion.

The novel pyridylacetonitrile derivatives of the general formula (I) according to the present invention can be produced by the process shown by the following reaction formula:

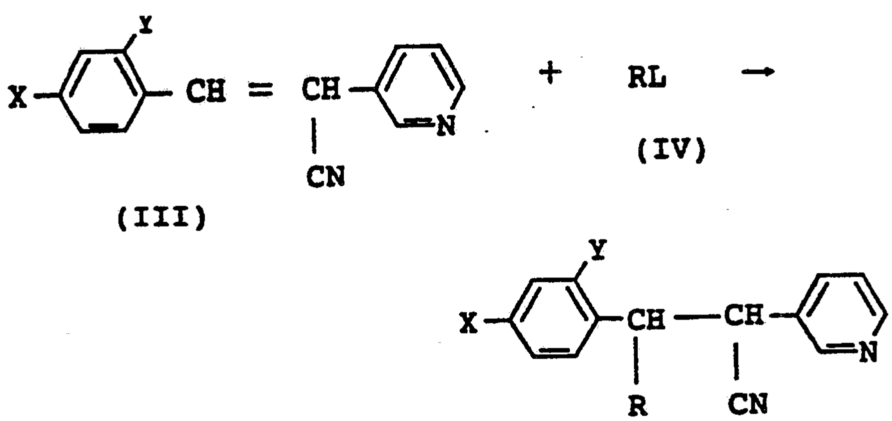

(III) (IV)

(I)

In the above reaction formula, X, Y, and R are the same as that defined above, and L represents, lithium, MgCℓ, MgBr or MgI.

Thus, the olefins represented by the general formula (III) are allowed to react with an organometallic compound represented by the general formula (IV).

The reaction is preferably carried out in the presence of an organic solvent at a temperature of from -80 °C to the boiling point of the solvent, more preferably from -5 °C to 20 °C. The reaction time preferably ranges from 1 to 24 hours. The preferred molar ratio of the compound (III) and the compound (IV) is 1:1 to 1:1.5.

The typical examples of the organic solvent are aliphatic hydrocarbons such as hexane, heptane or petroleum ether, aromatic hydrocarbons such as benzene, toluene or xylene, and ethers such as diethylether, diisopropylether, dioxane, tetrahydrofuran or diethyleneglycol dimethylether.

The olefins of the general formula (III) can be prepared from 3-pyridylacetonitrile by the methods as described, for example, in U.S. Patent 3,196,158 and Yakugaku Zasshi, vol. 89, pp. 188-193 (1969).

The desired product represented by the formula (I) can be isolated and purified in any conventional manner. The isomer of the product (I) can be separated by silica gel chromatography.

The compounds of the general formula (I) waherein X is a phenylethynyl group, can be also prepared by reacting the corresponding bromoderivatives (X in the general formula I is a bromine atom) with phenylacetylene in the presence of a catalyst such as palladium complex as described, for example, in Synthetic Example 3.

The metal complexes of the pyridylacetonitrile derivatives (I), which is represented by the general formula (II), can be prepared by reacting the compound of the general formula (I) with a metal salt in the presence of an organic solvent such as methanol, ethanol, propanol, chloroform, acetone, acetonitrile or diethylether, preferably methanol or ethanol. Thus, the compound (I) is dissolved in the organic solvent and the metal salt is then added, followed by stirring at room temperature. As a result, the desired metal complex can be obtained.

The compounds according to the present invention have two asymmetric carbon atoms and, therefore, four stereoisomers are present. These isomers and their mixture are within the scope of the present invention.

The pyridylacetonitrile derivatives or its acid salts and metal complexes in accordance with the present invention exhibit excellent fungicidal activity against various crop diseases, especially gray mold. The typical examples of such diseases are as follows:

Rice: Blast, Helminthosporium leaf spot, Sheath blight, "Bakanae" disease, Seedling blight
Wheat: Leaf rust, Stripe rust, Loose smut, Speckled leaf blotch, Spot blotch, Browing root rot, Powdery mildew
Potato: Late blight, Early blight, Black scurf
Soybean: Downy mildew, Cercospora leaf spot, Mycosphaerella sojae, Sclerotinia rot, Rust, Purple stain
Adzuki bean: Leaf spot, Rust, Leaf spot, Powdery mildew
Peanut: Brown leaf spot, Leaf spot, Sclerotinia rot
Tobacco: Brown spot, Black shank, Sclerotinia stem-rot, Sore shin, Damping-off, Frog-eye, Powdery mildew
Sugar beet: Downy mildew, Cercospora leaf spot, Damping-off
Tomato: Gray mold, Leaf mold, Late blight, Stem rot, Early blight, Cercospora leaf mold, Fusarium wilt, Damping-off
Eggplant: Gray mold, Verticillium wilt, Brown rot, Black rot, Powdery mildew, Leaf mold
Cucurbit: Downy mildew, Phytophthora rot, Gray mold, Sclerotinia rot, Scab, Anthracnose, Gummy stem wilt, Powdery mildew, Damping-off
Chinese cabbage: Downy mildew, Leaf spot, Sclerotinia rot, Clubroot
Onion: Downy mildew, Gray-mold neck rot. Phytophthora rot, Smudge
Lettuce: Downy mildew, Gray mold, Leaf spot, Stem rot
Strawberry: Gray mold, Powdery mildew, Leaf blight
Chrysanthemum: Rust, Powdery mildew
Cyclamen: Gray mold
Rose: Black spot, Powdery mildew
Citrus: Blue mold, Gray mold, Melanose, Scab
Apple: Rust, Alternaria leaf spot, Scab, Powdery mildew, Blossom blight
Pear: Rust, Black spot, Scab
Peach: Brown rot, Scab, Phomopsis rot, Leaf curl
Grape: Downy mildew, Gray mold, Leaf spot, Anthracnose, Ripe rot, Pestalotia disease, Rust, Dead arm, Powdery mildew

The effect of the compounds according to the present invention is not only preventive, but also curative. Moreover, the effect persists for long term and the phytotoxicity, especially growth suppression, caused by the fungicides in the present invention is extremely slight.

When the pyridylacetonitrile derivatives, an acid salt thereof or a metal complex thereof according to the present invention are used as effective ingredient of agricultural or horticultural fungicides, the compounds may be directly used. However, the present compounds can be formulated with solid carriers, liquid carriers, surfactants, and other adjuvants to form various forms of preparations such as emulsions, wettable powder, suspensions and granules. The present pyridylacetonitrile derivatives can be formulated as an effective ingredient into the preparations in an amount of 0.1% to 99.9% by weight, preferably 0.2% to 80% by weight.

Typical examples of the solid carriers usable in the formulation are finely divided powder or particles of

kaolin clay, attapulgite clay, bentonite, acid clay, pyrophyllite, talc, diatomeceous earth, calcite, and white carbon. Typical examples of the liquid carriers are aromatic hydrocarbons such as xylene and methyl naphthalene; alcohols such as isopropanol, ethylene glycol, and cellosolve; ketones such as acetone, cyclohexanone, and isophorone; vegetable oil such as bean oil and cotton seed oil; dimethyl sulfoxide; and acetonitrile.

Typical examples of the surfactants usable as an emulsifier, dispersing agent and/or wetting and spreading agent are anionic surfactants such as alkylsulfate salts, alkyl(or aryl)sulfonate salts, dialkylsulfosuccinate salts, polyolyoxyethylenealkylaryl ether phosphate salts, and naphthalene sulfonic acid formaldehyde condensates; and nonionic surfactants such as polyoxyethylenealkyl ether, polyoxyethylene polyoxypropylene block copolymers, and sorbitan fatty acid esters.

Typical examples of the adjuvants for formulation are lignin sulfonate, alginates, polyvinyl alcohol, gum arabic, carboxymethyl cellulose, and acidic isopropyl phosphate. It should also be noted that the effective component of the present fungicide can be also formulated into the preparations together with one or more agents such as various conventional insecticides, bactericides, fungicides, herbicides, plant growth regulators, miticides, nematocides, attractants, repellents, nutrients, fertilizers, and soil structure conditioning agents. Thus, these preparations are expected to exhibit various wide effects.

The present invention are now described in greater detail by reference to the following Synthetic Examples, Formulation Examples, and Test Examples, which are given here fore illustrative purpose only, and are not intended to limit the scope of the invention.

## SYNTHETIC EXAMPLE 1

Synthesis of 3-(2-chloro-4-fluorophenyl)-2-(3-pyridyl)pentanenitrile (Compound 12 shown in Table 1):

An anhydrous solution of 5.18 g (0.02 mole) of 2-(2-chloro-4-fluorophenyl)-1-(3-pyridyl)vinylcyanide in 50 ml of tetrahydrofuran was cooled to -20 °C. 30 ml of ethylmagnesium iodide solution in ether, which was prepared from 5.15 g (0.033 mole) of ethyl iodide and 0.72 g (0.03 gram/atom) of metal magnesium, was dropwise added thereto, controlling the reaction temperature below -10 °C. After the mixture was stirred at -20 °C for 2 hours and then at 0 °C for 3 hours, it was poured into the saturated aqueous solution of ammonium chloride. The organic solvent was distilled off and the residue was exstracted with ethyl acetate. The extract was dried over anhydrous magnesium sulfate and concentrated to give a 2.60 g of viscous oil. This oil was purified by silica gel chromatography using a 2:1 (by volume) mixture of n-hexane and ethyl acetate as an eluent. The Compound 12 was obtained in the form of a pale yellow oil (Yield = 2.07 g). The oil was a mixture of two diastereomers of the Compound 12. The mixture can be separated into the diastereomer A with larger $R_f$ value (mp. 84.7-86 °C) and the diastereomer B with smaller $R_f$ value (mp. 68.5-69 °C) by silica gel chromatography using a 2:1 (by volume) mixture of n-hexane and ethyl acetate as an eluent.

## SYNTHETIC EXAMPLE 2

Synthesis of 3-(2,4-dichlorophenyl)-2-(3-pyridyl)heptanenitrile (Compound 18 shown in Table 1):

An anhydrous solution of 5.5 g (0.02 mole) amount of 2-(2,4-dichlorophenyl)-1-(3-pyridyl)vinylcyanide in 30 ml of tetrahydrofuran was cooled to -l0 °C. A solution of butylmagnesium bromide in 20 ml of diethylether, which was prepared from 4.52 g (0.033 mole) of 1-bromobutane and 0.72 g (0.03 mole) of metal magnesium, was added thereto controlling the reaction temperature below 0 °C. After the mixture was stirred at room temperature for 3 hours, it was poured into a saturated aqueous solution of ammonium chloride. The organic solvent was distilled off and the residue was extracted with ethyl acetate. The extract was dried over anhydrous magnesium sulfate and concentrated to give viscous oil. This oil was subjected to silica gel chromatography using a 95:5 (by volume) mixture of chloroform and acetone as an eluent, and 3.10 g of the Compound 18 was obtained in the form of pale yellow oil.

## SYNTHETIC EXAMPLE 3

Synthesis of 3-(2-chloro-4-phenylethynylphenyl)-2-(3-pyridyl)pentanenitrile (Compound 33 shown in Table 1):

A mixture of 3.5 g (0.01 mole) of 3-(4-bromo-2-chlorophenyl)-2-(3-pyridyl)pentanenitrile, 1.6 g (0.015 mole) of phenylacetylene, 0.05 g of tetrakis(triphenyl phosphin)palladium (o), 0.1 g of copper iodine (I) and 0.2 g of triphenyl phosphin in 50 ml of triethylamine was stirred for 2 hours at 90°C under nitrogen atmosphere. The reaction mixture was conceantrated by evaporation and the residue was stirred with 150 ml of toluene. The thus obtained solution together with a solid phase still contained therein was washed with water and the organic layer was dried and evaporated to give an oily residue. It was purified by silica gel chromatography using a 2:1 (by volume) mixture of n-hexane and ethylacetate as an eluant. The yield of pure product was 4.0 g.

## SYNTHETIC EXAMPLE 4

Synthesis of the cupper complex of 3-(2,4-dichlorophenyl)-2-(3-pyridyl)pentanenitrile (Compound 34 shown in Table 2):

3.5 g (0.02 mole) of cupric chloride dihydrate ($CuC\ell_2 \cdot 2H_2O$) was dissolved in 10 ml of ethanol. A solution of 6.1 g (0.02 mole) of Compound 1 in 20 ml ethanol was dropwise added thereto with agitation at room temperature. The mixture was stirred for 2 hours at room temperature. The precipitated solid was separated by suction filtration and washed with a small amount of ethanol and air-dried. The yield was 7.4 g.

Various pyridylacetonitrile derivatives of the general formula (I) were prepared in the same manner as mentioned above. The results are shown in the following Table 1. The NMR spectrum was measured in deuterated chloroform with tetramethylsilan as an inner standard. In the NMR data, m, q, t, d and s represent multiplet, quartet, triplet, doublet and singlet, respectively. The typical examples of the metal complexes of the above mentioned general formula (II) are shown in Table 2.

Among the present compounds shown in Tables I and 2, Compounds 1, 2, 12, 13, 31, 33, 34 and 36 are more preferred.

Table 1

| Compound No. | Y | X | R | NMR Value | Melting Point or Index of Refraction |
|---|---|---|---|---|---|
| 1 | Cl | Cl | $C_2H_5$ | 8.50~8.20 ppm (m, 2H), 7.40~6.90 (m, 5H),4.20~3.40 ppm (m, 2H), 2.10~1.70 ppm (m,2H), 0.8 ppm (t, 3H, J=7Hz) | mp. 74~75°C |
| 2 | Cl | Cl | $C_3H_7$(n) | 8.50~8.25 ppm (m, 2H), 7.35~7.15 ppm (m,5H), 4.20~3.90 ppm (m, 1H), 3.80~3.60 ppm(m, 1H), 2.00~1.70 ppm (m, 2H), 1.40~1.15 ppm (m, 2H), 1.10~0.90 ppm (m, 3H) | $n_D^{20}$ 1.562 |
| 3 | H | Cl | $C_2H_5$ | 8.60~8.10 ppm (m, 2H), 7.40~6.80 ppm (m,6H), 4.10~3.80 ppm (m, 1H), 3.00~2.60 ppm(m, 1H), 2.05~1.60 ppm (m, 2H), 1.00~0.65 ppm (m, 3H) | $n_D^{20}$ 1.579 |
| 4 | H | Cl | $C_3H_7$(n) | 8.40~8.15 ppm (m, 2H), 7.30~6.75 ppm (m,6H), 4.10~3.80 ppm (m, 1H), 3.10~2.75 ppm(m, 1H), 2.10~1.60 ppm (m, 2H), 1.40~0.70 ppm (m, 5H) | $n_D^{20}$ 1.561 |
| 5 | Cl | Cl | $CH_3$ | 8.60~8.20 ppm (m, 2H), 7.40~6.80 ppm (m,5H), 4.25~4.10 ppm (m, 1H), 4.05~3.75 ppm(m, 1H), 1.50 ppm, 1.30 ppm (s, 3H) | $n_D^{20}$ 1.570 |

Table 1 (cont'd)

| Compound No. | Y | X | R | NMR Value | Melting Point or Index of Refraction |
|---|---|---|---|---|---|
| 6 | F | Cl | $C_2H_5$ | 8.55~8.30 ppm (m, 2H), 7.45~6.80 ppm (m,5H), 4.15 ppm (d, 1H, J=6Hz), 3.05 ppm (q,1H, J=7Hz), 2.15~1.65 ppm (m, 2H), 0.85 ppm (t, 3H, H=7Hz) | mp. 87~88°C |
| 7 | F | Cl | $CH_3$ | 8.60~8.30 ppm (m, 2H), 7.50~6.85 ppm (m,5H), 4.20~3.40 ppm (m, 2H), 1.60~1.35 ppm (m, 3H) | $n_D^{20}$ 1.548 |
| 8 | Cl | Cl | 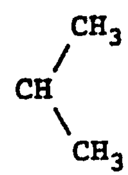 | 8.45~8.20 ppm (m, 2H), 7.70~7.00 , (m, 5H)4.55~4.05 ppm (m, 1H), 3.95~3.25 ppm (m,1H), 2.80~2.15 ppm (m, 1H),1.45~0.75 ppm (m, 6H) | $n_D^{20}$ 1.571 |
| 9 | F | Cl | $C_3H_7(n)$ | 8.50~8.30 ppm (m, 2H), 7.50~6.80 ppm (m, 5H), 4.20~4.10 ppm (m, 1H), 3.50~3.20 ppm (m, 1H), 2.10~1.60 ppm (m, 2H), 1.40~0.71 ppm (m, 5H) | |
| 10 | F | Cl | $C_4H_9(n)$ | 8.50~8.25 ppm (m, 2H), 7.40~6.85 ppm (m, 5H), 4.15~4.05 ppm (m, 1H), 3.50~3.25 ppm(m, 1H), 2.15~1.70 ppm (m, 2H), 1.50~1.00ppm (m, 4H), 0.95~0.75 ppm (m, 3H) | |

Table 1 (cont'd)

| Compound No. | Y | X | R | NMR Value | Melting Point or Index of Refraction |
|---|---|---|---|---|---|
| 11 | Cl | F | $CH_3$ | 8.55~8.20 ppm (m, 2H), 7.50~6.80 ppm (m, 5H), 4.20~3.70 ppm (m, 2H), 1.45 ppm (d, 3H, J=8Hz) | $n_D^{20}$ 1.555 |
| 12 | Cl | F | $C_2H_5$ | 8.50~8.20 ppm (m, 2H), 7.45~6.75 ppm (m, 5H), 4.20~4.10 ppm (m, 1H), 3.85~3.45 ppm (m, 1H), 2.10~1.65 ppm (m, 2H), 0.95~0.70 ppm (m, 3H) | |
| 13 | Cl | F | $C_3H_7$(n) | 8.40~8.20 ppm (m, 2H), 7.45~6.70 ppm (m, 5H), 4.20~4.10 ppm (m, 1H), 4.10~3.50 ppm (m, 1H), 2.10~1.65 ppm (m, 2H), 1.60~0.70 ppm (m, 5H) | $n_D^{20}$ 1.539 |
| 14 | Cl | F | $C_3H_7$(i) | 8.55~8.25 ppm (m, 2H), 7.65~7.05 ppm (m, 5H), 5.00~4.10 ppm (m, 1H), 4.00~3.30 ppm (m, 1H), 2.90~2.25 ppm (m, 1H), 1.45~0.80 ppm (m, 6H) | |
| 15 | F | F | $CH_3$ | 8.55~8.25 ppm (m, 2H), 7.50~6.50 ppm (m, 5H), 4.15~4.05 ppm (m, 1H), 3.80~3.35 ppm (m, 1H), 1.70~1.30 ppm (m, 3H) | mp. 82~83°C |

Table 1 (cont'd)

| Compound No. | Y | X | R | NMR Value | Melting Point or Index of Refraction |
|---|---|---|---|---|---|
| 16 | F | F | $C_2H_5$ | 8.55~8.35 ppm (m, 2H), 7.45~6.50 ppm (m, 5H), 4.25~4.15 ppm (m, 1H), 3.55~3.15 ppm (m, 1H), 2.05~1.60 ppm (m, 2H), 1.05~0.75 ppm (m, 3H) | |
| 17 | F | F | $C_3H_7$(n) | 8.50~8.25 ppm (m, 2H), 7.45~6.40 ppm (m, 5H), 4.20~4.10 ppm (m, 1H), 3.60~3.20 ppm (m, 1H), 2.05~1.65 ppm (m, 2H), 1.50~0.75 ppm (m, 5H) | |
| 18 | Cl | Cl | $C_4H_9$(n) | 8.45~8.20 ppm (m, 2H), 7.30~7.10 ppm (m, 5H), 4.15~4.05 ppm (m, 1H), 3.85~3.45 ppm (m, 1H), 2.10~1.65 ppm (m, 2H), 1.50~1.00 ppm (m, 4H), 0.95~0.75 ppm (m, 3H) | |
| 19 | Cl | H | $C_2H_5$ | 8.55~8.20 ppm (m, 2H), 7.30~7.00 ppm (m, 6H), 4.20~4.10 ppm (m, 1H), 3.90~3.50 ppm (m, 1H), 2.15~1.65 ppm (m, 2H), 0.95~0.75 ppm (m, 3H) | |
| 20 | $CF_3$ | Cl | $C_2H_5$ | 8.50~8.25 ppm (m, 2H), 7.55~7.00 ppm (m, 5H), 4.05 ppm (d, 1H, J=8Hz), 3.60~3.20 ppm (m, 1H), 2.10~1.50 ppm (m, 2H), 0.75 ppm (t, 3H, J=6Hz) | |

Table 1 (cont'd)

| Compound No. | Y | X | R | NMR Value | Melting Point or Index of Refraction |
|---|---|---|---|---|---|
| 21 | $CF_3$ | Cl | $nC_3H_7$ | 8.50~8.25 ppm (m, 2H), 7.55~7.00 ppm (m, 5H), 4.00 ppm (d, 1H, J=8Hz), 3.65~3.30 ppm (m, 1H), 2.00~1.55 ppm (m, 2H), 1.50~0.60 ppm (m, 2H+3H) | |
| 22 | $CH_3$ | Cl | $C_2H_5$ | 8.50~8.25 ppm (m, 2H), 7.30~6.55 ppm (m, 5H), 4.05 ppm (d, 1H, J=8Hz), 3.35~3.00 ppm (m, 1H), 2.05~1.55 ppm (m, 2H), 1.85 ppm (s, 3H), 0.75 ppm (t, 3H, J=6Hz) | |
| 23 | $CH_3$ | Cl | $nC_3H_7$ | 8.55~8.25 ppm (m, 2H), 7.35~7.00 ppm (m, 5H), 4.00 ppm (d, 1H, J=8Hz), 3.40, 3.15 ppm (q, 1H, J=6Hz), 2.00~1.60 ppm (m, 2H), 1.85 ppm (s, 3H), 1.35~0.70 ppm (m, 2H+3H) | |
| 24 | H | F | $isoC_4H_9$ | 8.40~8.10 ppm (m, 2H), 7.25~6.80 ppm (m, 6H), 4.05 ppm (d, 1H, J=8Hz), 3.20~2.85 ppm (m, 1H), 2.10~0.80 ppm (m, 9H) | |
| 25 | H | F | 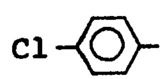 | 8.45~8.25 ppm (m, 2H), 7.40~6.70 ppm (m, 10H), 4.50 ppm (d, 1H, J=8Hz), 4.25 ppm (d, 1H, J=8Hz) | |

Table 1 (cont'd)

| Compound No. | Y | X | R | NMR Value | Melting Point or Index of Refraction |
|---|---|---|---|---|---|
| 26 | Cl | Br | $CH_3$ | 8.50~8.20 ppm (m, 2H), 7.35~6.90 ppm (m, 5H), 4.20~4.05 ppm (m, 1H), 4.00~3.75 ppm (m, 1H), 1.40 ppm (d, 3H, J=7Hz) | |
| 27 | Cl | Br | $C_2H_5$ | 8.50~8.25 ppm (m, 2H), 7.40~7.00 ppm (m, 5H), 4.20 ppm (d, 1H, J=8Hz), 3.80~3.45 ppm (m, 1H), 2.15~1.65 ppm (m, 2H), 0.80 ppm (t, 3H, J=6Hz) | |
| 28 | H | Br | $CH_3$ | 8.50~8.30 ppm (m, 2H), 7.40~6.80 ppm (m, 5H), 4.00 ppm (d, 1H, J=8Hz), 3.40~2.95 ppm (m, 1H), 1.45 ppm (d, 3H, J=8Hz) | |
| 29 | H | Br | $C_2H_5$ | 8.45~8.20 ppm (m, 2H), 7.35~6.75 ppm (m, 5H), 4.10 ppm (d, 1H, J=7Hz), 3.00~2.65 ppm (m, 1H), 2.10~1.60 ppm (m, 2H), 0.80 ppm (t, 3H, J=7Hz) | |
| 30 | H | 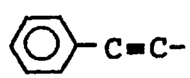 | $CH_3$ | 8.50~8.25 ppm (m, 2H), 7.40~6.70 ppm (m, 11H), 4.00~3.90 ppm (m, 1H), 3.40~3.00 ppm (m, 1H), 1.40 ppm (d, 3H, J=6Hz) | |

Table 1 (cont'd)

| Compound No. | Y | X | R | NMR Value | Melting Point or Index of Refraction |
|---|---|---|---|---|---|
| 31 | H | 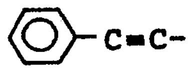 | $C_2H_5$ | 8.40~8.25 ppm (m, 2H), 7.35~6.70 ppm (m, 11H), 4.10~4.00 ppm (m, 1H), 3.05~2.60 ppm (m, 1H), 2.10~1.60 ppm (m, 2H), 0.80 ppm (t, 3H, J=7Hz) | |
| 32 | Cl | 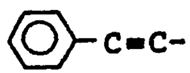 | $CH_3$ | 8.50~8.20 ppm (m, 2H), 7.40~7.10 ppm (m, 11H), 4.30~4.10 ppm (m, 1H), 4.10~3.50 ppm (m, 1H), 1.40 ppm (d, 3H, J=6Hz) | |
| 33 | Cl | 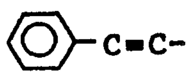 | $C_2H_5$ | 8.55~8.30 ppm (m, 2H), 7.70~7.05 ppm (m, 11H), 4.20~4.00 ppm (m, 1H), 3.90~3.40 ppm (m, 1H), 2.20~1.65 ppm (m, 2H), 0.80 ppm (t, 3H, J=7Hz) | |

Table 2

| Compound No. | Chemical Structure | Melting Point(°C) |
|---|---|---|
| 34 | $CuCl_2$ complex of Compound 1 (m = 2, n = 2) | 208-210 |
| 35 | $CuCl_2$ complex of Compound 2(m = 2, n = 2) | 155-157 |
| 36 | $CuCl_2$ complex of Compound 12 (m = 2, n = 2) | 210-213 |

Formulation examples containing a compound according to the present invention are given below.

## FORMULATION EXAMPLE 1

Power fungicides having the following compositions were prepared by mixing the ingredients listed below at room temperature.

| Ingredient | Parts by weight |
|---|---|
| Compound listed in Table 1 or 2 | 3 |
| Clay | 40 |
| Talc | 57 |

## FORMULATION EXAMPLE 2

Wettable powder fungicides having the following compositions were prepared by mixing the ingredients listed below at room temperature.

| Ingredient | Parts by weight |
|---|---|
| Compound listed in Table 1 or 2 | 25 |
| Polyoxyethylene alkylarylether | 9 |
| White carbon | 16 |
| Talc | 50 |

The wettable powder fungicide is thus obtained were evaluated in the Test Examples below.

## FORMULATION EXAMPLE 3

Emulsion fungicides having the following compositions were prepared by mixing the ingredients listed below at room temperature.

| Ingredient | Weight in 100 ml |
|---|---|
| Compound listed in Table 1 or 2<br>Sorpol 2680® (manufactured by Toho Kagaku Kogyo K.K.)<br>Xylene | 20 g<br>10 g<br>balance |

The agricultural or horticultural fungicide according to the present invention can be preferably used in an amount of 1 to 500 g, in terms of an active ingredient, per 10 are (a) when the fungicide is sprayed to the field having growing crops thereon and 0.1 to 5 kg, in terms of an acitve ingredient, per 10 are (a), when the fungicide is applied into the soil. It should be, of course, noted that the amount of the active ingredient depends upon the kinds of crops, diseases, and damages, seasons, weather, and the preparation forms of the fungicides.

The agricultural or horticultural fungicides according to the present invention were evaluated in the following Test Examples. The reference compounds A to G, which were used in each test, are shown in Table 12.

## TEST EXAMPLE 1

Evaluation test of antifungal activity against powdery mildew of cucumber

A sample solution containing a predetermined amount of the active compound was sprayed on cucumber seedlings (variety: Sagami Hanjiro) at 2 leaf stage in a pot with the volume of 200 liter/10 a and the treated cucumber seedlings were air-dried in a room.

After air drying, the treated cucumber seedlings was allowed to stand in a green house to naturally infect with the spores of Sphaerotheca fuliginea.

After 14 days, the degree of the infection was calculated according to the following criteria. Five tests were carried out in each run and ten leaves were evaluated.

wherein

n: Number of leaves in each infection degree

f: Index of infection degree

N: Number of leaves examined.

| Index of infection degree | Infected area (%) |
|---|---|
| 0 (No infection) | 0 |
| 1 (Low infection) | 1-25 |
| 3 (Medium infection) | 26-50 |
| 5 (Large infection) | 51-100 |

The results are shown in Table 3.

## Table 3

### Antifungal activity against powdery mildew of cucumber

| Compound No. | Concetr. (a.i., ppm) | No. of leaves in each infection degree 0 | 1 | 3 | 5 | % of Infected leaves | Infection degree | Chemical injury |
|---|---|---|---|---|---|---|---|---|
| 1 | 20 | 10 | 0 | 0 | 0 | 0 | 0 | - |
| 2 | 20 | 10 | 0 | 0 | 0 | 0 | 0 | - |
| 3 | 20 | 9 | 1 | 0 | 0 | 10 | 2 | - |
| 4 | 20 | 8 | 2 | 0 | 0 | 20 | 4 | - |
| 5 | 20 | 10 | 0 | 0 | 0 | 0 | 0 | - |
| 6 | 20 | 10 | 0 | 0 | 0 | 0 | 0 | - |
| 7 | 20 | 10 | 0 | 0 | 0 | 0 | 0 | - |
| 8 | 20 | 10 | 0 | 0 | 0 | 0 | 0 | - |
| 9 | 20 | 9 | 1 | 0 | 0 | 10 | 2 | - |
| 10 | 20 | 9 | 1 | 0 | 0 | 10 | 2 | - |
| 11 | 20 | 10 | 0 | 0 | 0 | 0 | 0 | - |
| 12 | 20 | 10 | 0 | 0 | 0 | 0 | 0 | - |

Table 3 (cont'd)

| Compound No. | Concetr. (a.i., ppm) | No. of leaves in each infection degree 0 | 1 | 3 | 5 | % of Infected leaves | Infection degree | Chemical injury |
|---|---|---|---|---|---|---|---|---|
| 13 | 20 | 10 | 0 | 0 | 0 | 0 | 0 | - |
| 14 | 20 | 10 | 0 | 0 | 0 | 0 | 0 | - |
| 15 | 20 | 10 | 0 | 0 | 0 | 0 | 0 | - |
| 16 | 20 | 10 | 0 | 0 | 0 | 0 | 0 | - |
| 17 | 20 | 9 | 1 | 0 | 0 | 10 | 2 | - |
| 18 | 20 | 9 | 1 | 0 | 0 | 10 | 2 | - |
| 19 | 20 | 10 | 0 | 0 | 0 | 0 | 0 | - |
| 20 | 20 | 10 | 0 | 0 | 0 | 0 | 0 | - |
| 21 | 20 | 10 | 0 | 0 | 0 | 0 | 0 | - |
| 22 | 20 | 10 | 0 | 0 | 0 | 0 | 0 | - |
| 23 | 20 | 10 | 0 | 0 | 0 | 0 | 0 | - |
| 24 | 20 | 9 | 1 | 0 | 0 | 10 | 2 | - |
| 25 | 20 | 10 | 0 | 0 | 0 | 0 | 0 | - |
| 28 | 20 | 6 | 2 | 2 | 0 | 40 | 16 | - |
| 29 | 20 | 6 | 3 | 1 | 0 | 40 | 12 | - |
| 30 | 20 | 7 | 3 | 0 | 0 | 30 | 6 | - |
| 31 | 20 | 10 | 0 | 0 | 0 | 0 | 0 | - |
| 32 | 20 | 10 | 0 | 0 | 0 | 0 | 0 | - |
| 33 | 20 | 10 | 0 | 0 | 0 | 0 | 0 | - |
| 34 | 20 | 9 | 1 | 0 | 0 | 10 | 2 | - |
| B | 20 | 10 | 0 | 0 | 0 | 0 | 0 | - |
| Control | - | 0 | 0 | 2 | 8 | 100 | 92 | |

Note: The mark "-" in Chemical injury shows that phytotoxicity was extremely slight.

TEST EXAMPLE 2

Evaluation test of antifungal activity against leaf rust of wheat

A spore suspension previously prepared in such a way that 20 spores of Puccinia recondita f. sp. tritici were present in a visual area of 100 magnification microscope was sprayed on wheat seedlings (variety: Norin #61) at 2 leaf stage in a pot.

Immediately after inoculation, the sprayed wheat seedlings were allowed to stand under a dark condition at a temperature of 23° C in an R.H. of 100% for 28 hours. After 28 hours, the wheat seedlings were transferred to a green house and, after 3 days from the inocuration, a sample fungicide solution containing a predetermined amount of the active compound was sprayed on the wheat seedlings with the volume of 200 liter/10 a. After 10 days from the inoculation, the degree of the infection was calculated as follows. Five tests were carried out in each run.

$$\text{Degree of infection} = \frac{\Sigma\, n\, f}{5\, N} \times 100$$

wherein n: Number of leaves in each infection degree
f: Index of infection degree
N: Number of leaves examined

| Index of infection degree | Infected area (%) |
|---|---|
| 0 (No infection) | 0 |
| 1 (Low infection) | 1-25 |
| 3 (Medium infection) | 26-50 |
| 5 (Large infection) | 51-100 |

The results are shown in Table 4.

## Table 4

### Antifungal activity against leaf rust of wheat

| Compound No. | Concetr. (a.i., ppm) | No. of leaves in each infection degree 0 | 1 | 3 | 5 | % of Infected leaves | Infection degree | Chemical injury |
|---|---|---|---|---|---|---|---|---|
| 1 | 50 | 10 | 0 | 0 | 0 | 0 | 0 | - |
| 2 | 50 | 10 | 0 | 0 | 0 | 0 | 0 | - |
| 5 | 50 | 10 | 0 | 0 | 0 | 0 | 0 | - |
| 6 | 50 | 10 | 0 | 0 | 0 | 0 | 0 | - |
| 7 | 50 | 10 | 0 | 0 | 0 | 0 | 0 | - |
| 8 | 50 | 10 | 0 | 0 | 0 | 0 | 0 | - |
| 9 | 50 | 10 | 0 | 0 | 0 | 0 | 0 | - |
| 11 | 50 | 10 | 0 | 0 | 0 | 0 | 0 | - |
| 12 | 50 | 10 | 0 | 0 | 0 | 0 | 0 | - |
| 13 | 50 | 10 | 0 | 0 | 0 | 0 | 0 | - |
| 14 | 50 | 10 | 0 | 0 | 0 | 0 | 0 | - |
| 15 | 50 | 10 | 0 | 0 | 0 | 0 | 0 | - |

## Table 4 (cont'd)

| Compound No. | Concetr. (a.i., ppm) | No. of leaves in each infection degree | | | | % of Infected leaves | Infection degree | Chemical injury |
|---|---|---|---|---|---|---|---|---|
| | | 0 | 1 | 3 | 5 | | | |
| 16 | 50 | 10 | 0 | 0 | 0 | 0 | 0 | - |
| 19 | 50 | 10 | 0 | 0 | 0 | 0 | 0 | - |
| 20 | 50 | 10 | 0 | 0 | 0 | 0 | 0 | - |
| 22 | 50 | 10 | 0 | 0 | 0 | 0 | 0 | - |
| 25 | 50 | 10 | 0 | 0 | 0 | 0 | 0 | - |
| 31 | 50 | 10 | 0 | 0 | 0 | 0 | 0 | - |
| 32 | 50 | 10 | 0 | 0 | 0 | 0 | 0 | - |
| 33 | 50 | 10 | 0 | 0 | 0 | 0 | 0 | - |
| 35 | 50 | 9 | 1 | 0 | 0 | 10 | 2 | - |
| 36 | 50 | 10 | 0 | 0 | 0 | 0 | 0 | - |
| C | 50 | 10 | 0 | 0 | 0 | 0 | 0 | - |
| Control | - | 0 | 0 | 0 | 10 | 100 | 100 | |

## TEST EXAMPLE 3

### Evaluation test of antifungal activity against gray mold of cucumber

A sample solution containing a predetermined amount of the active compound was sprayed on cucumber seedlings (variety: Suhyoo) at 3 leaf stage in a pot at a volume of 200 liter/10 a and the treated cucumber seedlings were air-dried for 24 hours in a room.

After air drying, a spore suspension was prepared in such a way that 100 conidia of Botrytis cinerea were present in a visual area of a 100 magnification microscope and a commercially available yeast extract (1% amount) and a commercially available glucose (10% amount) were added thereto. The resultant suspension was inoculated by spraying. Immediately, the sprayed cucumber were allowed to stand under a dark condition at a temperature of 22°C and an R.H. of 100%. After 3 days, the degree of the infection was measured. The protection value was calculated as follows. Eight tests were carried out in each run.

$$\text{Degree of infection} = \frac{\sum n f}{5 N} \times 100$$

wherein
n: Number of leaves in each infection degree
f: Index of infection degree
N: Number of leaves examined

| Index of infection degree | Infected area (%) |
|---|---|
| 0 (No infection) | 0 |
| 1 (Low infection) | 1-25 |
| 3 (Medium infection) | 26-50 |
| 5 (Large infection) | 51-100 |

The results are shown in Table 5.

## Table 5

Antifungal activity against gray mold of cucumber

| Compound No. | Concentration (a.i., ppm) | Degree of infection | Chemical injury |
|---|---|---|---|
| 1 | 250 | 10.0 | - |
| 2 | 250 | 13.0 | - |
| 3 | 250 | 45.0 | - |
| 4 | 250 | 50.0 | - |
| 5 | 250 | 12.0 | - |
| 6 | 250 | 25.0 | - |
| 7 | 250 | 25.0 | - |
| 8 | 250 | 25.0 | - |
| 9 | 250 | 26.5 | - |
| 10 | 250 | 28.0 | - |
| 11 | 250 | 10.0 | - |
| 12 | 250 | 9.5 | - |
| 13 | 250 | 10.0 | - |
| 14 | 250 | 15.5 | - |
| 15 | 250 | 25.0 | - |
| 16 | 250 | 25.0 | - |
| 17 | 250 | 25.0 | - |
| 18 | 250 | 25.0 | - |
| 19 | 250 | 25.0 | - |
| 20 | 250 | 50.0 | - |
| 21 | 250 | 42.0 | - |

Table 5 (cont'd)

| Compound No. | Concentration (a.i., ppm) | Degree of infection | Chemical injury |
|---|---|---|---|
| 23 | 250 | 53.0 | - |
| 25 | 250 | 53.0 | - |
| 28 | 250 | 58.0 | - |
| 30 | 250 | 28.0 | - |
| 31 | 250 | 28.0 | - |
| 32 | 250 | 30.0 | - |
| 33 | 250 | 41.0 | - |
| 34 | 250 | 26.0 | - |
| 36 | 250 | 10.0 | - |
| A | 250 | 28.0 | ++growth suppression |
| D | 250 | 13.0 | - |
| Control | - | 100 | |

## TEST EXAMPLE 4

Evaluation test of antifungal activity against Sclerotinia rot of kidney bean

A 20 ml sample solution containing a predetermined concentration of the active compound was sprayed on 10 cotyledons of a kidney bean (variety: Masterpiece), which were dried in a room. After drying, the mycelia of Sclerotinia sclerotiorum grown on a PDA culture medium was inoculated after punching with a cork borer. The incubation was carried out at a temperature of 28° C and an RH of 100% for 4 days. The infected area was measured. Ten tests were carried out in each run.

Controlled value $(1\text{-}S/S_0)\times 100$

wherein

S: average infected area of leaves treated by an active compound

$S_0$: average infected area of no-treated leaves

The results are shown in Table 6.

Table 6

| Antifungal activity against Sclerotinia rot of kidney bean | | | | |
|---|---|---|---|---|
| Compound No. | Concentration (a.i., ppm) | Average infected area | Controlled value | Chemical injury |
| | | (%) | (%) | |
| 1 | 500 | 5 | 95 | - |
| 2 | 500 | 5 | 95 | - |
| 5 | 500 | 8 | 92 | - |
| 11 | 500 | 5 | 95 | - |
| 12 | 500 | 5 | 95 | - |
| 13 | 500 | 5 | 95 | - |
| 14 | 500 | 8 | 92 | - |
| 20 | 500 | 15 | 85 | - |
| 30 | 500 | 20 | 80 | - |
| 32 | 500 | 12 | 88 | - |
| A | 500 | 40 | 60 | - |
| E | 500 | 20 | 80 | - |
| Control | - | 100 | 0 | |

## TEST EXAMPLE 5

### Evaluation test of antifungal effect against rice blast disease

A sample solution containing a predetermined amount of the active solution was sprayed on rice seedlings (variety: Jyukkoku) at 3 leaf stage in a pot with the volume of 200 liter/10 a and the treated rice seedlings were air-dried.

After air drying, a spore suspension previously prepared in such a way that 40 conidia of rice blast fungus (Pyricularia oryzae) were present in a visual area of a 100 magnification microscope was inoculated by spraying. Immediately, the sprayed rice seedlings were allowed to stand under a dark condition of a temperature of 23°C and an R.H. of 100%. After 48 hour inoculation, the rice seedlings were transferred to a green house and, after 10 days from the inocuration, the degree of the infection was measured. The protection value was calculated as follows. Three tests were carried out in each run.

$$\text{Degree of infection} = \frac{\Sigma\, n f}{4\, N} \times 100$$

wherein
n: Number of leaves in each infection degree
f: Index of infection degree
N: Number of leaves examined

| Index of infection degree | Number of Lesion per leaf |
|---|---|
| 0 | 0 |
| 1 | 1 |
| 2 | 2-5 |
| 3 | 6-10 |
| 4 | 11 or more |

The results are shown in Table 7.

Table 7

| Antifungal activity against rice blast | | | | | | | | | | |
|---|---|---|---|---|---|---|---|---|---|---|
| Compound No. | Concetr. (a.i., ppm) | No. of infected leaves | No. of leaves in each infection degree | | | | | % of Infected leaves | Infection degree | Chemical injury |
| | | | 0 | 1 | 2 | 3 | 4 | | | |
| 1 | 500 | 206 | 145 | 48 | 13 | 0 | 0 | 29.6 | 9.0 | - |
| F | 500 | 185 | 128 | 45 | 12 | 0 | 0 | 30.8 | 9.3 | - |
| G | 500 | 180 | 151 | 28 | 1 | 0 | 0 | 16.1 | 4.2 | - |
| Control | - | 204 | 0 | 0 | 71 | 81 | 42 | 100 | 72.7 | |

## TEST EXAMPLE 6

Evaluation test of antifungal activity against sheath blight of rice

A sample solution containing a predetermined concentration of the active compound was sprayed on cotyledons of a kidney bean (variety: Masterpiece), which were dried in a room.

After air drying the mycelia of Rhizoctonia solani IA grown on a PDA culture was inoculated after punching with a 6 mmØ cork borer. The incubation was carried out at a temperature of 28°C and an R.H. of 100% for 3 days under dark conditions. The infected area was measured. Five tests were carried out in each run.

The results are shown in Table 8.

**Table 8**

**Antifungal activity against sheath blight of rice**

| Compound No. | Concentration (a.i., ppm) | Controlled value (%) | Chemical injury |
|---|---|---|---|
| 1 | 500 | 100 | - |
| 2 | 500 | 100 | - |
| 3 | 500 | 90 | - |
| 4 | 500 | 100 | - |
| 5 | 500 | 95 | - |
| 6 | 500 | 95 | - |
| 7 | 500 | 95 | - |

**Table 8**

| Compound No. | Concentration (a.i., ppm) | Degree of infection | Chemical injury |
|---|---|---|---|
| 8 | 500 | 95 | - |
| 9 | 500 | 93 | - |
| 10 | 500 | 90 | - |
| 11 | 500 | 100 | - |
| 12 | 500 | 100 | - |
| 13 | 500 | 100 | - |
| 14 | 500 | 100 | - |
| 15 | 500 | 95 | - |
| 16 | 500 | 95 | - |
| 17 | 500 | 95 | - |
| 18 | 500 | 95 | - |
| 19 | 500 | 95 | - |
| 24 | 500 | 93 | - |
| 30 | 500 | 100 | - |
| 32 | 500 | 94 | - |
| 33 | 500 | 90 | - |
| 36 | 500 | 100 | - |
| B | 500 | 75 | - |
| Control | - | 0 | |

## TEST EXAMPLE 7

Antifungal activity against Botrytis cinerea by agar dilution method

20 ml of a potato-agar medium containing a predetermined concentration of each active ingredient was poured into a petri dish. After solidifying, the tip portion of the flora of Botrytis cinerea previously incubated on a plate culture medium, which was punched with a cork borer having a diameter of 6 mm, was inoculated on the culture medium containing the active ingredient. The culture medium thus obtained was allowed to stand in a room with constant temperature for predetermined days. Thereafter, the growth of

mycelium of the microorganism was measured and the concentration for 90% inhibition of the growth ($EC_{90}$) was determined. Four tests were carried out in each run.

The tests for microorganisms were carried out at a temperature of 25 °C for 5 days. The results are shown in Table 9.

Table 9

| Antifungal activity against Botrytis cinerea | |
|---|---|
| Compound No. | $EC_{90}$ (µg/ml) |
| 1 | 1.4 |
| 2 | 1.6 |
| 5 | 1.5 |
| 11 | 1.5 |
| 12 | 0.9 |
| 13 | 1.2 |
| A | 10.9 |

## TEST EXAMPLE 8

Evaluation test of activity persistence against gray mold of egg plant

A sample solution containing a predetermined amount of the active compound was sprayed on egg plant seedlings (variety: Senryo #2) at 4-leaf stage in a pot with the volume of 200 liter/10 a on one day, four days, seven days before inoculation, and the treated egg plant seedlings were administered in a green house. The inoculation and the measurement of the degree of infection were carried out in the same way as described in Test Example 3.

The results are shown in Table 10.

Table 10

| Persistence of activity against gray mold of egg plant | | | | | |
|---|---|---|---|---|---|
| Compound No. | Concentr. (a.i., ppm) | Infection degree | | | |
| | | 1* | 4* | 7* | 10* |
| 1 | 250 | 10.0 | 12.0 | 17.5 | 17.5 |
| 2 | 250 | 12.0 | 15.5 | 18.0 | 21.5 |
| 5 | 250 | 12.0 | 16.0 | 25.0 | 31.5 |
| 6 | 250 | 25.0 | 28.0 | 32.0 | 43.5 |
| 7 | 250 | 25.0 | 28.0 | 35.5 | 45.0 |
| 11 | 250 | 10.0 | 12.0 | 15.5 | 18.0 |
| 12 | 250 | 10.0 | 11.0 | 12.0 | 15.0 |
| 13 | 250 | 11.0 | 11.0 | 13.0 | 17.5 |
| A | 250 | 25.0 | 37.5 | 45.0 | 62.5 |
| D | 250 | 12.0 | 15.5 | 20.5 | 25.0 |
| Control | - | 100 | 100 | 100 | 100 |
| Note: "*" is stand for the number of days between the application and the inoculation | | | | | |

## TEST EXAMPLE 9

Evaluation test of curative activity against gray mold of egg plant

A second developed leaf of egg plant (variety: Senryo #12) was placed in a petri dish and the mycelia of Botrytis cinerea grown on a PDA culture medium was inoculated after punching with a cork borer on the center of the leaf. The incubation was carried out at 25°C under humid condition for 24 hours. After the confirmation of the disease development, a sample solution in 250 ppm was sprayed on the leaf at the volume rate of 20 ml per 8 plates. After air drying, the incubation was carried out at 25°C under humid condition. The diameter of the infected area was measured every day for 5 days after the application. Eight tests were carried out in each run.

The results are shown in Table 11.

Table 11

| Curative activity against gray mold of egg plant | | | | | | |
|---|---|---|---|---|---|---|
| Compound No. | Concentr. (a.i., ppm) | Diameter of lesion (mm) | | | | |
| | | 1* | 2* | 3* | 4* | 5* |
| 1 | 125 | 10 | 15 | 17 | 19 | 19 |
| 2 | 125 | 10 | 15 | 18 | 20 | 20 |
| 5 | 125 | 10 | 16 | 18 | 21 | 22 |
| 6 | 125 | 10 | 17 | 20 | 22 | 25 |
| 7 | 125 | 10 | 18 | 20 | 23 | 25 |
| 11 | 125 | 10 | 15 | 17 | 19 | 19 |
| 12 | 125 | 10 | 15 | 17 | 19 | 19 |
| 13 | 125 | 10 | 15 | 18 | 20 | 21 |
| A | 125 | 10 | 18 | 23 | 26 | 30 |
| E | 125 | 10 | 18 | 21 | 22 | 26 |
| Control | - | 10 | 22 | 31 | 43 | 55 |
| Note: "*" is stand for the number of days between the application and observation. | | | | | | |

Table 12

| Referance Compounds | | |
|---|---|---|
| Code | Chemical name | Brandname & source |
| A | 3-(2,4-dichlorophenyl)-2-(1-imidazolyl)hexanenitrile | JP-A-86-167668 |
| B | Validamycin A | Validacin from Takeda Chemical Industries, Ltd. |
| C | 1-(4-chlorophenoxy)-2,3-dimethyl-1-(1,2,4-triazol-1-yl)-2-butanone | Bayreton® from Bayer AG |
| D | N-(3′,5′-dichlorophenyl)-1,2-dimethylcyclopropane-1,2-dicarboximide | Sumisclex® from Sumitomo Chemical Industries, Limited |
| E | dimethyl-4,4′-o-phenylene-bis(3-thioalfanate) | Topsin® from Nisso |
| F | diisopropyl-1,3,-dithiolan-2-ylidene malonate | Fugione® from Nihon Nohyaku Co., Ltd. |
| G | 4,5,6,7-tetrachlorophthalide | Lafcide® from Kureha Chemical Industry Co., Ltd. |

From the results shown in the above test examples, it can be seen that the fungicides containing, as an effective ingredient, the comopunds according to the present invention exhibit excellent antifungal effect against broad range of crop diseases. When the present compounds are applied to crop fields, presistence can be exhibited. Further, the effect of the compounds according to the present invention is not only preventive, but also curative. Moreover, the phytotoxicity against crops, especially tomato, cucumber, egg plant, green pepper, root crops, etc., caused by the fungicides of the present inveniton is extremely slight.

## Claims

1. A pyridylacetonitrile derivative represented by the general formula (I), an acid salt thereof or a metal complex therof:

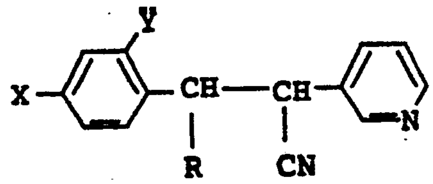

(I)

wherein X is a halogen atom or a phenylethynyl group, Y is a hydrogen atom, a halogen atom, a methyl group, or a trifluoromethyl group, R is a lower alkyl group or a phenyl group which may be substituted or unsubstituted with a halogen atom.

2. A pyridylacetonitrile derivative as claimed in Claim 1, wherein X is a chlorine atom, a fluorine atom or a phenylethynyl group.

3. A pyridylacetonitrile derivative as claimed in Claim 1, wherein Y is a chlorine atome or a fluorine atom.

4. A pyridylacetonitrile derivative as claimed in Claim 1, wherein R is an alkyl group having 1 to 4 carbon atoms.

5. A pyridylacetonitrile derivative as claimed in Claim 1, wherein said acid salt is derived from hydrochloric acid, hydrobromic acid, sulfuric acid, oxalic acid or sulfonic acid.

6. A pyridylacetonitrile derivative as claimed in Claim 1, wherein said complex is represented by the general formula (II):

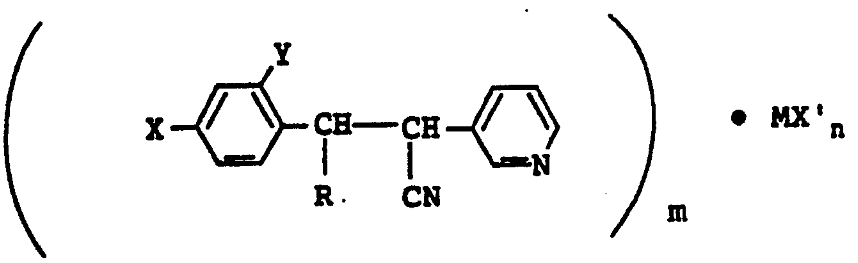

(II)

wherein X, Y and R are as defined in Claim 1, M is a metal atom, X′ is a counter ion, and n and m each represents integers of 1 to 4.

7. A pyridylacetonitrile derivative as claimed in Claim 6, wherein M in the general formula (II) is aluminum, manganese, cobalt, iron, nickel, cupper or zinc.

8. A pyridylacetonitrile derivative as claimed in Claim 6. wherein X′ in the general formula (II) is chloride, bromide, iodide, sulfate, phosphate, nitrate, carbonate or acetate ion.

9. A process for producing a pyridylacetonitrile derivative represented by the general formula (I), an acid salt thereof, or a metal complex thereof:

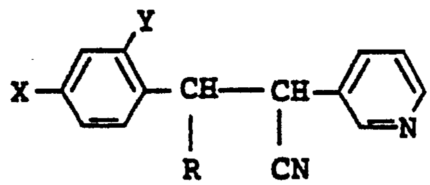

(I)

wherein X is a halogen atom or a phenylethynyl group, Y is a hydrogen atom, a halogen atom, a methyl group or a trifluoromethyl group, R is a lower alkyl group or a phenyl group which may be substituted or unsubstituted with a halogen atom, which comprises reacting a compound of the general formula (III)

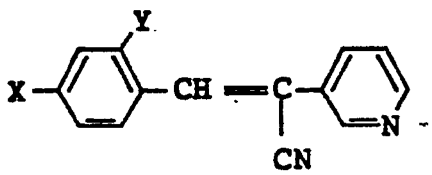

(III)

wherein X and Y are as defined in Claim 1, with a compound of general formula (IV)
RL (IV)
wherein R is as defined above, and L represents lithium, MgCl, MgBr or MgI.

10. A fungicide which comprises an effective amount of a pyridylacetonitrile derivative represented by the general formula (I), an acid salt thereof, or a metal complex thereof:

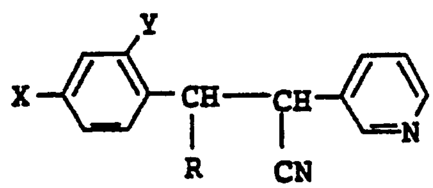

(I)

wherein X is a halogen atom or a phenylethynyl group, Y is a hydrogen atom, a halogen atom, a methyl group or a trifluoromethyl group, R is a lower alkyl group or a phenyl group which may be substituted or unsubstituted with a halogen atom.

11. A fungicide as claimed in Claim 10, wherein X is a chlorine atom, a fluorine atom or a phenylethynyl group, Y is a chlorine atom or a fluorine atom, R is an alkyl group having 1 to 4 carbon atoms.

12. A fungicide as claimed in Claim 10, wherein said acid salt is derived from hydrochloric acid, hydrobromic acid, sulfuric acid, oxalic acid or sulfonic acid.

13. A fungicide as claimed in Claim 10, wherein said metal complex is represented by the general formula (II):

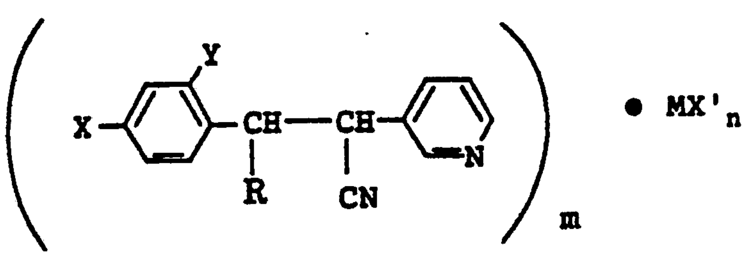

(II)

wherein X, Y, R are as defined in Claim 10 and M is a metal atom, X′ is a counter ion, and n and m each represents integers of 1 to 4.

14. A fungicide as claimed in Claim 13, wherein M in the general formula (II) is aluminum, manganese, cobalt, iron, nickel, cupper or zinc.

15. A fungicide as claimed in Claim 13, wherein X′ in the general formula (II) is chloride, bromide, iodide, sulfate, phosphate, nitrate, carbonate or acetate ion.